# EUROPÄISCHE PATENTANMELDUNG

(11) **EP 1 369 088 A1**
(43) Veröffentlichungstag der Anmeldung: **10.12.2003**
(21) Anmeldenummer: 03010249.5
(22) Anmeldetag: 07.05.2003
(51) Int. Cl.: A61B 17/74

(54) **Schenkelhalsschraube**

(30) Priorität: 05.06.2002 DE 20208922 U
(71) Anmelder: Stryker Trauma GmbH, 24232 Schönkirchen (DE)
(72) Erfinder: Krug, Florian, Dr. med., 22529 Hamburg (DE)
(74) Vertreter: Graalfs, Edo, Dipl.-Ing.

(57) **Zusammenfassung**

Schenkelhalsschraube mit einem Schaft (12) und einem Gewindeabschnitt (14), der selbstformend in den Femurkopf eindrehbar ist, dadurch gekennzeichnet, daß die Seele des Gewindeabschnitts (14) zumindest teilweise hohl ist.

## Beschreibung

Die Erfindung bezieht sich auf eine Schenkelhalsschraube nach dem Oberbegriff des Anspruchs 1.

Schenkelhalsschrauben werden verwendet bei Brüchen des Femurhalses, Femurkopfes und auch bei subtrochanteren Frakturen. Schenkelhalsschrauben werden üblicherweise zusammen mit einer sogenannten Pohl'schen Lasche oder mit einem Verriegelungsnagel kombiniert. Im letzteren Fall wird die Schenkelhalsschraube durch eine schräge Durchbohrung des Verriegelungsnagels im proximalen Bereich hindurchgeführt und in den Femurkopf eingeschraubt.

Eine Schenkelhalsschraube mit der zuletzt beschriebenen Anwendung ist etwa in EP 0 257 118 B1 offenbart. Die Besonderheit der dort beschriebenen Schenkelhalsschraube besteht darin, daß sie mit achsparallelen Nuten am Schaft versehen ist, so daß mit Hilfe eines in den Verriegelungsnagel eingeschraubten Verriegelungsstiftes eine Drehung der Schenkelhalsschraube verhindert, eine axiale Bewegung jedoch zugelassen wird.

Die bekannte Schenkelhalsschraube ist mit einem konischen selbst schneidenden Gewinde versehen. Die Flankenwinkel sind sehr klein, so daß eine relativ scharfe Gewindespitze geschaffen ist; die selbst schneidende Eigenschaft wird durch mindestens zwei achsparallele Nuten bewerkstelligt.

Es ist bekannt, beim Einsatz von Schenkelhalsschrauben den Femurhals und den Femurkopf im spongiosen Bereich vorzubohren. Hierdurch und durch die Ausbildung des Gewindes für die Schenkelhalsschraube findet eine deutliche Schwächung des Femurkopfes statt.

Der Erfindung liegt die Aufgabe zugrunde, eine Schenkelhalsschraube zu schaffen, die mit einem Gewinde versehen wird, das zu einer verringerten Schwächung des Femurkopfes führt.

Diese Aufgabe wird durch die Merkmale des Anspruchs 1 gelöst.

Bei der erfindungsgemäßen Schenkelhalsschraube ist die Seele des Gewindeabschnitts zumindest teilweise hohl. Hierdurch wird ein Gewinde geschaffen, das einem Korkenziehergewinde mit hohler Seele gleicht. Diese Maßnahme hat den Vorteil, daß einerseits ein derartiges Gewinde in der Lage ist, relativ leicht in die Spongiosa des Femurkopfes einzudringen, d. h. sich ein Muttergewinde zu formen, andererseits jedoch relativ viel Material stehen läßt, so daß nur eine minimale Schwächung des Femurkopfes bewirkt wird.

Nach einer Ausgestaltung der Erfindung kann der Gewindeabschnitt und der Schaft von einer gemeinsamen axialen Bohrung durchsetzt sein, die vorzugsweise über ihre gesamte Länge den gleichen Durchmesser aufweisen kann. Auf diese Weise ist es möglich, einen Führungsspieß für das Eindrehen der Schenkelhalsschraube zur Hilfe zu nehmen, um eine ausreichende Führung beim Einschrauben zu gewährleisten. Nach einer anderen Ausgestaltung der Erfindung weist der Gewindeabschnitt vorzugsweise ein Flachgewinde auf, das eine relativ große Profilbreite bei relativ geringer Steigung aufweisen kann. Dadurch wird ein Gewinde erhalten, das eine relativ hohe Tragkraft bereitstellt, die in jedem Falle ausreicht, die über den Femurkopf aufgebrachte Last auf den Femurschaft zu übertragen.

Nach einer anderen Ausgestaltung der Erfindung kann das Flachgewinde außen eine Fase aufweisen, die auf der Seite der Flanke geformt ist, welche zum freien Ende des Gewindeabschnitts hin weist. Die Fase erleichtert das Einschrauben des Gewindeabschnitts, ohne daß eine signifikante Schwächung der Tragkraft des Gewindeabschnitts bewirkt wird.

Bis auf das Eindrehende des Gewindeabschnitts weist dieses vorzugsweise einen konstanten Außendurchmesser auf. Vom freien Ende ausgehend kann der Gewindeabschnitt in seiner Profilbreite von einem minimalen Wert allmählich auf den konstanten Wert ansteigen. Diese Ausbildung erleichtert das Eindrehen des Gewindeabschnitts in den Femurkopf, insbesondere, wenn dieser nicht vorgebohrt wird. Vorzugsweise ist dabei das Eindrehende mit einer Spitze versehen, um den Eindrehvorgang weiter zu erleichtern.

Nach einer anderen Ausgestaltung der Erfindung entspricht der Außendurchmesser des Gewindeabschnitts annähernd dem Außendurchmesser des übrigen Schaftes der Schenkelhalsschraube.

Es ist von Vorteil, wenn der Gewindeabschnitt allmählich in den Schaft ausläuft. Dies kann dadurch geschehen, daß der Innendurchmesser des Gewindes allmählich bis zum Außendurchmesser des Schaftes vergrößert wird, beispielsweise schraubenlinienförmig. Auf diese Weise ist in dem Auslaufbereich ein Hohlkern geschaffen, der am Ende den Außendurchmesser des Schaftes aufweist.

Die Erfindung wird nachfolgend anhand eines Ausführungsbeispiels näher erläutert.
- Fig. 1: zeigt die Seitenansicht einer Schenkelhalsschraube nach der Erfindung.
- Fig. 2: zeigt einen Schnitt durch die Darstellung nach Fig. 1 entlang der Linie 2-2.
- Fig. 3: zeigt einen Schnitt durch einen Gewindegang eines Gewindeabschnitts einer erfindungsgemäßen Schenkelhalsschraube in abgewandelter Ausführung.

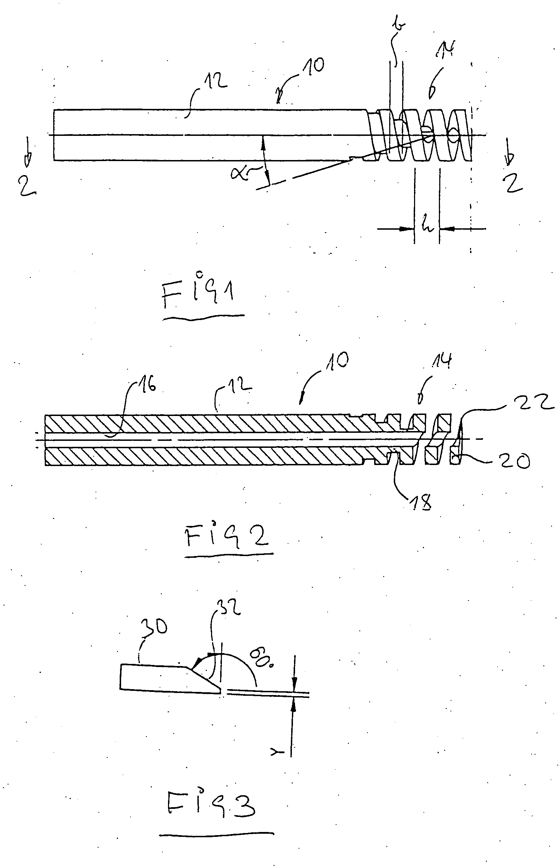

In den Fign. 1 und 2 ist eine Schenkelhalsschraube 10 zu erkennen, die einen glatten Schaft 12 und einen Gewindeabschnitt 14 aufweist. Der Gewindeabschnitt 14 weist ein sogenanntes Flachgewinde auf, wobei das Querschnittsprofil rechteckig ist. Die Steigung h ist relativ gering, wobei jedoch eine relativ hohe Profilbreite b vorgesehen ist.

Wie sich aus Fig. 2 ergibt, erstreckt sich eine Bohrung 16 durch die gesamte Schenkelhalsschraube 10, d. h. auch durch den Gewindeabschnitt 14. Dadurch ergibt sich in einem Bereich des Gewindeabschnitts 14, wie in Fig. 2 am rechten Ende erkennbar, eine hohle Seele für das Gewinde, die etwa in der Mitte des Gewindeabschnitts 14 in einen Hohlkern 18 übergeht. Der Hohlkern erweitert seinen Außendurchmesser schraubenlinienförmig bis zum Außendurchmesser des Schaftes 12, der im übrigen gleich ist dem Außendurchmesser des Gewindeabschnitts 14. Der Auslaufwinkel α beträgt z. B. annähernd 11°.

Der erste Gang 22 des Gewindeabschnitts 14 am freien Ende geht von einer Spitze 22 aus, deren Profilbreite zu Beginn noch sehr klein ist und allmählich zunimmt und nach einem Gang die Breite der übrigen Gewindegänge hat.

Ein typischer Durchmesser für die Gewindeschraube ist z. B. annähernd 12 mm. Die Profilbreite kann zwischen 1 mm und 5 mm betragen und die Steigerung kann zwischen 6 mm und 10 mm liegen. Die Länge des Gewindeabschnitts 14 beträgt z. B. 40 mm. Der Durchmesser der Bohrung 16 beträgt z. B. 3,5 mm.

Es ist zu erkennen, daß der Gewindeabschnitt 14 trotz der hohlen Seele relativ stabil ist und mithin hohe Tragkräfte aufzunehmen in der Lage ist. Andererseits kann er relativ leicht in die Spongiosa eines Femurkopfes eingedreht werden und läßt relativ viel Material stehen, wodurch die Schwächung des Femurkopfes minimal wird.

In Fig. 3 ist ein Querschnittsprofil 30 eines Gewindegangs für eine Schenkelhalsschraube angedeutet, welches im Gegensatz zu der Schenkelhalsschraube nach Fign. 1 und 2 eine Fase 32 an der Außenseite hat, so daß sich nur eine geringe Profilbreite y ergibt. Der Winkel der Fase beträgt 60°, wobei die Fase zum freien Ende des Gewindeabschnitts hin weist. Die Profilbreite kann zwischen 0,1 mm und 1 mm verändert sein.

## Patentansprüche

1. Schenkelhalsschraube mit einem Schaft und einem Gewindeabschnitt, der selbstformend in den Femurkopf eindrehbar ist, **dadurch gekennzeichnet, daß** die Seele des Gewindeabschnitts (14) zumindest teilweise hohl ist.

2. Schenkelhalsschraube nach Anspruch 1, **dadurch gekennzeichnet, daß** Gewindeabschnitt (14) und Schaft (12) von einer gemeinsamen axialen Durchbohrung (16) durchsetzt sind.

3. Schenkelhalsschraube nach Anspruch 1 oder 2, **dadurch gekennzeichnet, daß** der Gewindeabschnitt (14) ein Flachgewinde aufweist.

4. Schenkelhalsschraube nach Anspruch 3, **dadurch gekennzeichnet, daß** der Steigungswinkel relativ klein ist.

5. Schenkelhalsschraube nach Anspruch 3 oder 4, **dadurch gekennzeichnet, daß** die Flanken außen eine Fase aufweisen.

6. Schenkelhalsschraube nach Anspruch 5, **dadurch gekennzeichnet, daß** die Fase (32) auf der Seite der Flanke geformt ist, welche dem freien Ende des Gewindeabschnitts zugewandt ist.

7. Schenkelhalsschraube nach Anspruch 5 oder 6, **dadurch gekennzeichnet, daß** der Winkel der Fase (32) 60° beträgt.

8. Schenkelhalsschraube nach einem der Ansprüche 5 bis 7, **dadurch gekennzeichnet, daß** die Profilbreite außen einen Bruchteil der Profilbreite innen ist.

9. Schenkelhalsschraube nach einem der Ansprüche 1 bis 8, **dadurch gekennzeichnet, daß** vom freien Ende des Gewindeabschnitts (14) ausgehend die Profilbreite von einem minimalen Wert allmählich auf einen konstanten Wert ansteigt.

10. Schenkelhalsschraube nach Anspruch 9, **dadurch gekennzeichnet, daß** der Anstieg über einen Gewindegang erfolgt.

11. Schenkelhalsschraube nach einem der Ansprüche 1 bis 10, **dadurch gekennzeichnet, daß** das Ende (22) des zum freien Ende hin auslaufenden Gewindegangs des Gewindeabschnitts (14) vom Ende her betrachtet spitz ausläuft.

12. Schenkelhalsschraube nach einem der Ansprüche 1 bis 11, **dadurch gekennzeichnet, daß** der Außendurchmesser des Gewindeabschnitts (14) annähernd gleich dem Außendurchmesser des Schafts (12) ist.

13. Schenkelhalsschraube nach einem der Ansprüche 1 bis 12, **dadurch gekennzeichnet, daß** nahe dem Schaft (12) der Gewindeabschnitt (14) einen Hohlkern (18) aufweist.

14. Schenkelhalsschraube nach Anspruch 13, **dadurch gekennzeichnet, daß** der Hohlkern sich schraubenlinienförmig in Richtung Schaft vergrößert und in den Schaft (12) ausläuft.

15. Schenkelhalsschraube nach Anspruch 14, **dadurch gekennzeichnet, daß** der Gewindeauslauf in einem Winkel von 11° erfolgt.

16. Schenkelhalsschraube nach einem der Ansprüche 1 bis 15, **dadurch gekennzeichnet, daß** das Verhältnis von Durchmesser der Bohrung (16) zum Außendurchmesser des Schaftes (12) mindestens 1:3 ist, vorzugsweise 1:3,5.

17. Schenkelhalsschraube nach einem der Ansprüche 1 bis 16, **dadurch gekennzeichnet, daß** die Profilbreite 1 mm bis 5 mm beträgt.

18. Schenkelhalsschraube nach einem der Ansprüche 5 bis 17, **dadurch gekennzeichnet, daß** die Profilbreite an der Außenseite der Fase (32) 0,5 mm bis 1 mm beträgt.
